# EUROPEAN PATENT SPECIFICATION

(11) **EP 3 169 412 B1**
(45) Date of publication and mention of the grant of the patent: **24.04.2019**
(21) Application number: 15730218.3
(22) Date of filing: 12.06.2015
(51) Int. Cl.: A63B 23/18, A63B 21/00, A61M 16/00

(54) **RESPIRATORY THERAPY ASSEMBLIES**
ATEMTHERAPIEANORDNUNGEN
ENSEMBLES DE THÉRAPIE RESPIRATOIRE

(30) Priority: 19.07.2014 GB 201412867
(43) Date of publication of application: 24.05.2017
(73) Proprietor: Smiths Medical International Limited, Ashford, Kent TN25 4BF (GB)
(72) Inventor: BENNETT, Paul James Leslie, Marston Moretaine Bedfordshire MK43 0AQ (GB); BURCHELL, Robert James, Baldock Hertfordshire SG7 6DA (GB)
(74) Representative: Flint, Jonathan McNeill
(86) International application number: PCT/GB2015/000178
(87) International publication number: WO 2016/012740

(56) References cited:
- US-A- 4 533 137
- US-A1- 2012 272 956
- US-A1- 2013 319 416

## Description

This invention relates to respiratory therapy assemblies of the kind including a respiratory therapy device of the kind having a mechanism through which the patient exhales arranged to produce a variable oscillating resistance, to breathing through the device.

Patients with respiratory system diseases (such as asthma, COPD, cystic fibrosis or the like) may suffer from hyper-secretion of mucus as a prominent pathophysiological feature. Moreover, those patients with hyper-secretion often also have impaired mucus transport. This imbalance between mucus transport and secretion results in mucus retention in the respiratory system.

Vibratory respiratory positive expiratory pressure (V-PEP) or oscillatory PEP (OPEP) devices are modern devices for applying chest physiotherapy. These devices apply chest physiotherapy by providing an alternating resistance to flow and have been found to be particularly effective. One example of such apparatus is sold under the trade mark Acapella (a registered trade mark of Smiths Medical) by Smiths Medical and is described in US6581598, US6776159, US7059324 and US7699054. US2013/0319416 describes a device with a randomly variable resistance to breathing. Other vibratory respiratory therapy apparatus is available, such as "Quake" manufactured by Thayer, "AeroPEP" manufactured by Monaghan, "TheraPEP" manufactured by Smiths Medical, "IPV Percussionator" manufactured by Percussionaire Corp, and the "Flutter" and "Lung Flute" devices, amongst others. These devices are used by patients who suffer from mucus hyper-secretions and retention to help them clear the secretions from their lungs. The Acapella O-PEP device combines the principles of low-frequency oscillation and positive expiratory pressure by employing a counterweighted lever and magnet to produce oscillatory positive pressures during expiration. This generated oscillating positive pressure works by mechanically reducing the viscoelasticity of the sputum by breaking down the bonds of mucus macromolecules which, in turn, enhances mucociliary clearance. It is thought that the effectiveness of mucus clearance is dependent on maximizing the net shear forces applied to sputum. It is also important that the shear forces are applied in the appropriate direction to move mucus out of the lungs. It is believed that if the flow acceleration during the inhalation phase could be limited it would minimise the retreat of mucus that has been moved out during the expiratory phase.

It is an object of the present invention to provide an alternative respiratory therapy device.

The problem posed is solved according to the invention by the technical features of claim 1.

According to one aspect of the present invention there is provided a respiratory assembly of the above-specified kind, characterised in that the assembly includes a variable restrictor in an inhalation path of the assembly, and that the variable restrictor includes a slider the position of which is adjusted by the user to vary the restriction to inhalation flow that provides a variable resistance to inhalation flow.

The restrictor may be provided by a unit separate from the therapy device and connect to a patient port of the therapy device, the restrictor including a mouthpiece through which the patient breathes and the inhalation path through which the air flows during inhalation. The restrictor may include a housing with two apertures side by side, one aperture being arranged to connect with the respiratory therapy device and including an expiratory non-return valve, the other aperture opening to atmosphere and including both a manually-displaceable member arranged to obstruct inspiratory flow through the aperture and an inspiratory non-return valve, and both apertures opening into a mouthpiece in the restrictor.

According to another aspect of the present invention there is provided a variable restrictor for use in a respiratory therapy assembly according to the above one aspect of the present invention.

A respiratory therapy assembly according to the present invention will now be described, by way of example, with reference to the accompanying drawings, in which:
- Figure 1: is a perspective view of the assembly;
- Figure 2: is a perspective exploded view of the therapy device forming a part of the assembly;
- Figure 3: is a front perspective view of the variable inspiratory restrictor of the assembly;
- Figure 4: is a rear perspective view of the restrictor shown in Figure 3;
- Figure 5: is a front view of the restrictor with the mouthpiece and inspiratory non-return valve removed; and
- Figure 6: is a schematic diagram showing the circuit of the restrictor.

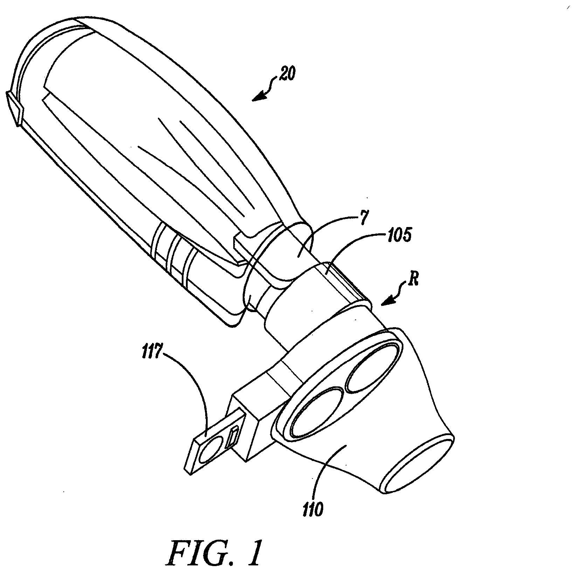

With reference first to Figure 1 there is shown an assembly of a conventional expiratory respiratory therapy device 20 and a novel restrictor R connected to the breathing inlet 7 of the therapy device. The restrictor R, which is described in detail later, serves to provide a variable restriction to inhalation but provides no significant restriction to exhalation.

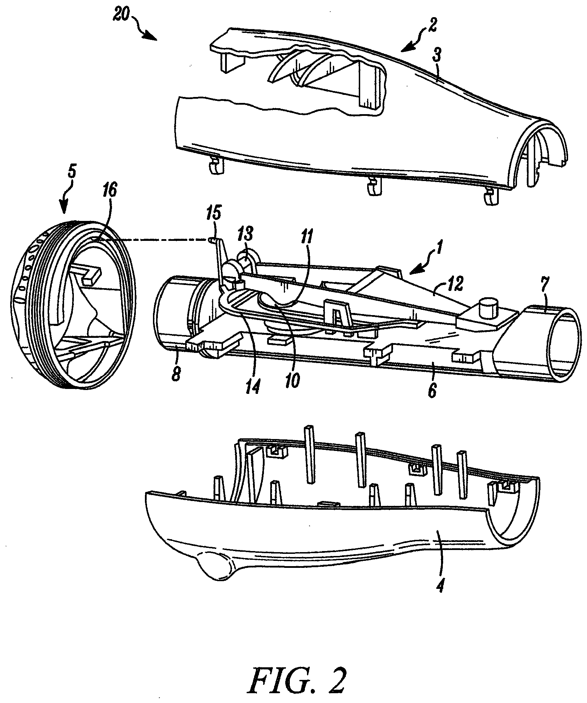

With reference now to Figure 2, the therapy device 20 is a conventional "Acapella" O-PEP device comprising a rocker assembly 1 contained within an outer housing 2 provided by an upper part 3 and a lower part 4 of substantially semi-cylindrical shape. The device 20 is completed by an adjustable dial 5 of circular section. The rocker assembly 1 includes an air flow tube 6 with a patient port or breathing inlet 7 at one end and an inspiratory inlet 8 at the opposite end including a one-way valve (not shown) that allows air to flow into the air flow tube 6 but prevents air flowing out through the inspiratory inlet. The air flow tube 6 has an outlet opening 10 with a non-linear profile that is opened and closed by a conical valve element 11 mounted on a rocker arm 12 pivoted midway along its length about a transverse axis. The air flow tube 6 and housing 2 provide a structure with which the rocker arm 12 is mounted. At its far end, remote from the breathing inlet 7, the rocker arm 12 carries an iron pin 13 that interacts with the magnetic field produced by a permanent magnet (not visible) mounted on an adjustable support frame 14. The magnet arrangement is such that, when the patient is not breathing through the device, the far end of the rocker arm 12 is held down such that its valve element 11 is also held down in sealing engagement with the outlet opening 10. A cam follower projection 15 at one end of the support frame 14 locates in a cam slot 16 in the dial 5 such that, by rotating the dial, the support frame 14, with its magnet, can be moved up or down to alter the strength of the magnetic field interacting with the iron pin 13. The dial 5 enables the frequency of operation and the resistance to flow of air through the device to be adjusted. Other O-PEP devices may have different setting arrangements for adjusting operation of the device and may be graduated in other ways, such as in frequency.

The inspiratory or inhalation path through the device 20 provided by the inspiratory inlet 8 is, in fact, not needed in the present assembly because the variable restrictor R provides an inhalation path in the manner described below.

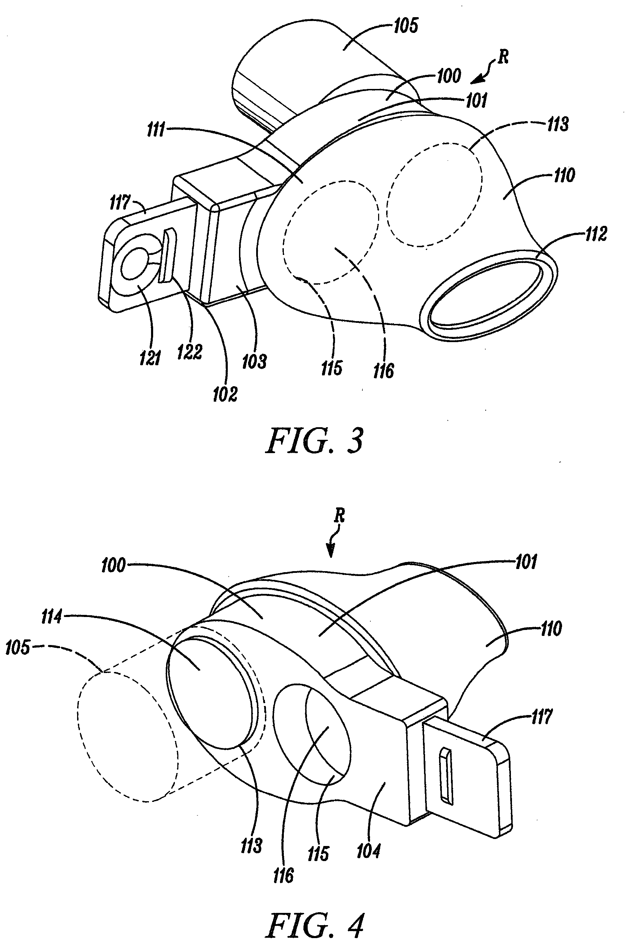

With reference now to Figures 3 to 6, the restrictor R includes a plastics housing 100 with an oval formation 101 at its right-hand end and a rectangular formation 102 at its left-hand end. The housing 100 has a flat front surface 103 and a flat rear surface 104. A tubular coupling 105 projects from the rear surface 104 of the oval formation 101. The internal diameter of the coupling 105 is chosen to form a secure air-tight fit on the outside of the breathing inlet 7 of the therapy device 20. The restrictor R also includes a mouthpiece 110 that is oval in section and tapers along its length from a forward, relatively wide end 111, which is attached around the front surface 103 of the oval formation 101. The opposite end 112 of the mouthpiece 110 is less wide and is open, being adapted to be received in the mouth of the patient. The mouthpiece 110 could be of a slightly deformable material to allow a better sealing fit with the patient's mouth. The housing 100 has a first circular, expiratory aperture 113 extending through it between the front and rear surfaces 103 and 104 in alignment with the centre of the coupling 105. The aperture 113 supports an expiratory non-return valve 114, such as a flexible flap valve, which allows air to flow from the restrictor R into the therapy device 20 but prevents any substantial flow in the opposite direction. The housing 100 also has a second circular, inspiratory aperture 115 extending through the housing and located to one side of the first aperture 113 within the oval formation 101. This aperture 115 is covered by an inspiratory non-return valve 116, such as a flexible flap valve, that allows air to be drawn through the aperture into the mouthpiece 110 but substantially prevents air flow in the opposite direction. The restrictor R also includes a slider 117 in the form of a generally rectangular plate extending laterally of the housing 20 through a slot 118 in its left-hand end so that the left-hand end 119 of the slider projects from the housing and its right-hand end 120 extends within the housing across the bore of the inspiratory aperture 115. The projecting, left-hand end of the slider 117 has a finger grip on its forward surface in the form a shallow depression 121 and, to its right, a stop in the form of a raised lip 122 that limits the extent by which the slider can be pushed inwardly into the housing 100. The slider 117 also has a similar stop (not visible) within the housing 100 that limits how far out the slider can be pulled. The right-hand end 120 of the slider 117 is curved with a semi-circular shape of the same diameter as the inspiratory aperture 115. When the slider 117 is pushed full into the housing 20 its forward, right-hand end 120 engages the curved side of the bore through the aperture 115 substantially to block all gas flow through the aperture. When the slider 117 is pulled fully outwardly, its forward, right-hand end 120 is fully displaced out of the bore through the inspiratory aperture 115 to allow a maximum free air flow through the aperture. It is, however, not essential that the slider 117 be movable between a fully closed and fully open position, only that the slider be movable to allow a variation in the restriction to inspiratory flow through the aperture 115 to suit the particular patient or range of patients. The slider 117 is preferably marked in some way to allow the patient to move it to a desired position suitable for the therapy.

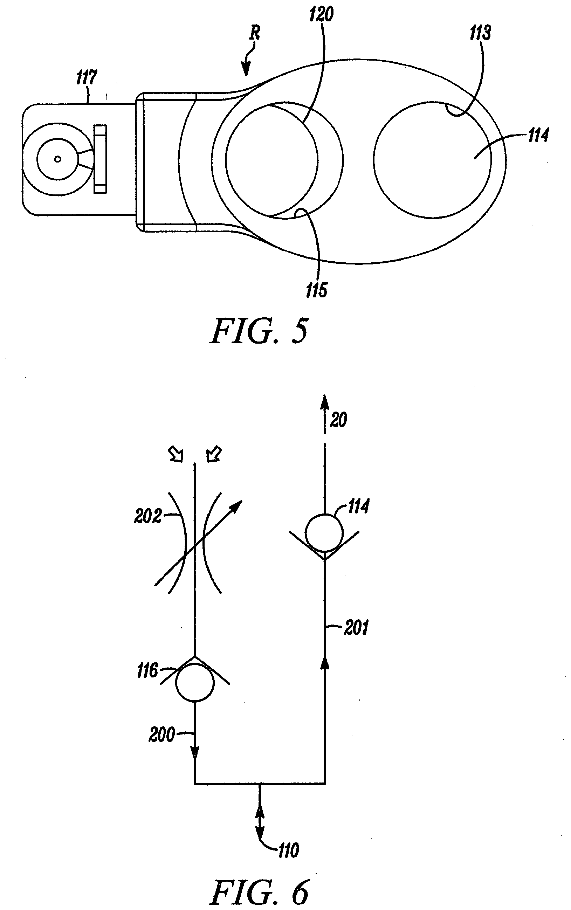

Figure 6 illustrates the circuit provided by the restrictor R, which has an inhalation path 200 and an exhalation path 201 that both connect with the mouthpiece 110. The inhalation path 200 includes a variable flow restrictor 202 provided by the slider 117 movable across the aperture 115. This connects with the mouthpiece 110 via the inspiratory non-return valve 116. The exhalation path 201 connects the mouthpiece 110 to the inlet of the therapy device 20 via the expiratory non-return valve 114.

It will be appreciated that there are many alternative arrangements by which a variable resistance to inhalation flow could be provided, such as, for example, by an arrangement including a rotatable plate with a slot of varying width or with apertures of different diameters or by an arrangement involving compressing and releasing a clamp on a compliant flow tube.

When the patient inhales through the mouthpiece 110 air is drawn along the inhalation path 200 through the restrictor R, the non-return valve 114 in the exhalation path 201 preventing air being drawn in through the aperture 113 from the therapy device 20. When the patient exhales, the non-return valve 116 in the inhalation path 200 closes, preventing any air flowing out along this path. Instead, the expiratory pressure is applied to the breathing inlet 7 of the therapy device 20 and from there via the flow tube 6 to the underside of the valve element 11 on the rocker arm 12. This causes the valve element 11 to be lifted up out of the opening 10 against the magnetic attraction, thereby allowing air to flow out to atmosphere. The opening 10 has a non-linear profile, which causes the effective discharge area to increase as the far end of the rocker arm 12 lifts, thereby allowing the arm to fall back down and close the opening. As long as the user keeps applying sufficient expiratory pressure, the rocker arm 12 will rise and fall repeatedly as the opening 10 is opened and closed, causing a vibratory, alternating or oscillating interruption to expiratory breath flow through the device. Further information about the construction and operation of the device can be found in US6581598.

The user adjusts the settings of the position of the slider 117 in the restrictor R and the setting of the dial 5 controlling frequency and exhalation resistance to achieve maximum therapeutic benefit for the particular user according to his lung compliance and resistance. The settings are preferably such as to maximise the shear forces applied to sputum in a direction tending to drive sputum out of the lungs compared with the forces in the opposite direction.

The present invention overcomes the problem of existing expiratory therapy devices in that they do not have provision to adjust the restriction to inspiratory flow, thereby either providing too high a resistance to flow for weaker patients or too low a resistance to flow for patients that would benefit from a higher inspiratory resistance. The present invention has the additional advantage that the restrictor can be used to provide a variable resistance to inspiratory flow with conventional respiratory therapy devices.

## Claims

1. A respiratory therapy assembly including a respiratory therapy device (20) of the kind having a mechanism (10, 11) through which the patient exhales arranged to produce a variable oscillating resistance to breathing through the device, **characterised in that** the assembly includes a variable restrictor (R) in an inhalation path (200) of the assembly, wherein the variable restrictor includes a slider (117) movable along its length relative to the inhalation path (200) to vary the obstruction to flow along the inhalation path (200) provided by the slider (117).

2. A respiratory therapy assembly according to Claim 1, **characterised in that** the restrictor (R) is provided by a unit separate from the therapy device (20) and connected to a patient port (7) of the therapy device (20), and that the restrictor (R) includes a mouthpiece (110) through which the patient breathes and the inhalation path (200) through which the air flows during inhalation.

3. A respiratory therapy assembly according to Claim 1 or 2, **characterised in that** the restrictor (R) includes a housing (100) with two apertures (113 and 115) side by side, that one aperture (113) is arranged to connect with the respiratory therapy device (20) and includes an expiratory non-return valve (114), that the other aperture (115) opens to atmosphere and includes both a manually-displaceable member (117) arranged to obstruct inspiratory flow through the aperture (115) and an inspiratory non-return valve (116), and that both apertures (113 and 115) open into a mouthpiece (110) in the restrictor (R).

## Patentansprüche

1. Atemtherapieanordnung mit einer Atemtherapievorrichtung (20) der Art mit einem Mechanismus (10, 11), durch den der Patient ausatmet, der dazu beschaffen ist, einen variablen Oszillationswiderstand gegen die Atmung durch die Vorrichtung hindurch zu erzeugen, **dadurch gekennzeichnet, dass** die Anordnung einen variablen Durchflussbegrenzer (R) in einem Einatmungspfad (200) der Anordnung umfasst, wobei der variable Durchflussbegrenzer einen Schieber (117) umfasst, der entlang seiner Länge relativ zum Einatmungspfad (200) beweglich ist, um die Behinderung gegen die Strömung entlang des Einatmungspfades (200), die durch den Schieber (117) bereitgestellt wird, zu verändern.

2. Atemtherapieanordnung nach Anspruch 1, **dadurch gekennzeichnet, dass** der Durchflussbegrenzer (R) durch eine von der Therapievorrichtung (20) separate Einheit bereitgestellt ist und mit einem Patientenkanal (7) der Therapievorrichtung (20) verbunden ist, und dass der Durchflussbegrenzer (R) ein Mundstück (110), durch das der Patient atmet, und den Einatmungspfad (200), durch den die Luft während des Einatmens strömt, umfasst.

3. Atemtherapieanordnung nach Anspruch 1 oder 2, **dadurch gekennzeichnet, dass** der Durchflussbegrenzer (R) ein Gehäuse (100) mit zwei Öffnungen (113 und 115) nebeneinander umfasst, dass eine Öffnung (113) dazu beschaffen ist, mit der Atemtherapievorrichtung (20) zu verbinden, und ein Ausatmungsrückschlagventil (114) umfasst, dass die andere Öffnung (115) in die Atmosphäre mündet und sowohl ein manuell verschiebbares Element (117), das dazu beschaffen ist, die Einatmungsströmung durch die Öffnung (115) hindurch zu behindern, als auch ein Einatmungsrückschlagventil (116) umfasst, und dass beide Öffnungen (113 und 115) in ein Mundstück (110) im Durchflussbegrenzer (R) münden.

## Revendications

1. Ensemble de thérapie respiratoire comprenant un dispositif de thérapie respiratoire (20) du type comportant un mécanisme (10, 11) à travers lequel le patient exhale, agencé pour produire une résistance oscillante variable à la respiration à travers le dispositif,
**caractérisé en ce que** l'ensemble comprend un régulateur variable (R) dans un trajet d'inhalation (200) de l'ensemble, dans lequel
le régulateur variable comprend un coulisseau (117) mobile le long de sa longueur par rapport au trajet d'inhalation (200) pour varier l'obstruction pour s'écouler le long du trajet d'inhalation (200) prévu par le coulisseau (117).

2. Ensemble de thérapie respiratoire selon la revendication 1, **caractérisé en ce que** le régulateur (R) est fourni par une unité distincte du dispositif de thérapie (20) et est connecté à un port patient (7) du dispositif de thérapie (20), et **en ce que** le régulateur (R) comprend un embout buccal (110) à travers lequel le patient respire et le trajet d'inhalation (200) à travers lequel l'air circule pendant l'inhalation.

3. Ensemble de thérapie respiratoire selon la revendication 1 ou 2, **caractérisé en ce que** le régulateur (R) comprend un boîtier (100) avec deux ouvertures (113 et 115) côte à côte, **en ce qu'**une ouverture (113) est agencée pour se connecter au dispositif de thérapie respiratoire (20) et comprend un clapet anti-retour expiratoire (114), **en ce que** l'autre ouverture (115) s'ouvre sur l'atmosphère et comprend à la fois un élément pouvant être déplacé manuellement (117) conçu pour obstruer le flux inspiratoire à travers l'ouverture (115) et un clapet anti-retour inspiratoire (116), et **en ce que** les deux ouvertures (113 et 115) s'ouvrent dans un embout buccal (110) dans le régulateur (R).
